(19) Europäisches Patentamt

European Patent Office

Office européen des brevets

(11) **EP 1 061 361 A1**

(12) **EUROPEAN PATENT APPLICATION**

(43) Date of publication:
**20.12.2000 Bulletin 2000/51**

(51) Int. Cl.⁷: **G01N 27/24**, G01N 27/22,
G01N 33/46, G01R 27/26

(21) Application number: **99830322.6**

(22) Date of filing: **27.05.1999**

(84) Designated Contracting States:
**AT BE CH CY DE DK ES FI FR GB GR IE IT LI LU MC NL PT SE**
Designated Extension States:
**AL LT LV MK RO SI**

(71) Applicant:
**CEO Centro di Eccellenza Optronica
50125 Arcetri, Firenze (IT)**

(72) Inventor: **Rossetti, Francesco
52020 Faella, Pian di Sco', Arezzo (IT)**

(74) Representative:
**Mannucci, Michele et al
Ufficio Tecnico Ing.A. Mannucci,
Via della Scala 4
50123 Firenze (IT)**

(54) **Device and method for capacitively detecting defects in wood**

(57)    The device for detecting defects in a dielectric material, particularly wood, comprises at least one capacitive sensor (1), means for placing said capacitive sensor next to the dielectric material (M), and a detection circuit for detecting the presence of defects in the dielectric material according to the variations of the value of the dielectric constant detected by the sensor.

Fig. 4

EP 1 061 361 A1

## Description

The present invention relates to a device and a method for detecting defects in dielectric materials, particularly in wood.

In the industries which produce wooden articles, there is a known problem consisting in the presence, in this natural material, of defects and irregularities which must be identified for the purpose of rejecting those portions of the material that cannot be used in industrial production.

At the present time, this problem is tackled in an inefficient way, by inspection of the material with the naked eye and manual marking of the defective areas of the material which must be rejected. The disadvantages of this method are evident: the procedure is not only slow and expensive, owing to its labor-intensive nature, but also unreliable, since it is virtually impossible to detect the presence of internal defects, only external defects or those which appear on the surface being detectable.

The object of the present invention is to provide a procedure and a device which overcome the disadvantages of conventional methods. More particularly, the object of the invention is to provide a device which can rapidly, accurately and reliably identify defects in dielectric materials, particularly in wood.

The object of the present invention is also to provide a device which can automate the phase of defect identification.

A particular object of the invention is to provide a non-invasive investigative device and method capable of rapidly and reliably identifying the defects present in wood.

Without giving a precise description of its microscopic structure, it may be stated that dry wood Consists of thin micro-channels, with air inside them, having walls of organic material and external diameters of approximately 10 micrometres. In addition to this "fine" spatial composition, wood is also characterized by a macroscopic structure which is clearly visible to the naked eye. The macroscopic structure comprises defects such as knots, resin pockets, rot, cracks, and others.

The device and the method of investigation must be capable of finding average values of the "fine" structure, distinguishing the "coarse" structure and recognizing the crucial defects which make certain portions of wood material unusable for industrial production.

The invention is based primarily on the observation of the fact that wood is a dielectric, regardless of the defects which are present. The dielectric constant $\varepsilon$ which characterizes it is related locally to the following parameters:

- the chemical composition
- the density
- the water content
- the temperature.

For the sake of simplicity it may be assumed that the dielectric constant depends on the quantity of charges which are influenced by the external electromagnetic fields and on the polarizability of the molecules to which they belong. Variations in the chemical composition and in the density of the material always accompany the defects which are present, and consequently their presence can be related to the variations of the local dielectric constant of the wood.

From the preceding argument, it is clear that the method and device according to the present invention can be applied not only in the field of wood, but also more generally whenever it is necessary to carry out a non-invasive investigation to detect the presence of defects in a dielectric material whose dielectric constant varies locally in the presence of the defects to be detected.

Essentially, the invention relates to a device for detecting defects in a dielectric material, particularly wood, by the detection of the variation of the local dielectric constant of the material; the device according to the invention comprises at least one capacitive sensor, means for placing the capacitive sensor next to the dielectric material, and a detection circuit for detecting the presence of defects in the material according to the variations of the value of the dielectric constant detected by the sensor.

It is known that in a capacitor with flat parallel faces the capacity depends on the dielectric constant $\varepsilon$ of the material present between the electrodes, according to the formula

$$C = \varepsilon \, \frac{A}{d}$$

where A is the area of the electrodes and d is the distance between them. Additionally, a capacitor has an impedance Z given by:

$$Z = \frac{1}{j\omega C} = \frac{d}{j\omega A \varepsilon}$$

where $\bar{\omega}$ is the frequency of the electromagnetic field. The invention is based on the idea of using a capacitive sensor consisting in practice of the electrodes of a capacitor, of introducing the material to be investigated (particularly a sheet of wood) into the electromagnetic field between the electrodes, and of detecting the presence of defects from a variation of the local dielectric constant and consequently of the overall impedance of the sensor, which varies as a result of the variation of the local dielectric constant in the wood.

By making the wood (usually in the form of a panel or plank) slide between the electrodes of a capacitor, or in front of two adjacent electrodes, the variations of the local dielectric constant of the wood modifies, as a result of the relative movement, the impedance of the capacitor, and consequently the operating frequency of an electronic oscillator inserted into a circuit in which the capacitor is connected. The wavelength of the electromagnetic field which is used is much greater than the dimensions of the defects, and therefore the electromagnetic radiation "sees" the wood inserted into the electromagnetic field between the electrodes as though it were homogeneous and the value of the dielectric constant which is measured is found as the average for this volume. In this way the choice of the dimensions of the electrodes defines the sampling volume, which will be large enough to provide an average value for the "fine" structure and comparable with the defects which are to be identified.

In practice, in a first embodiment of the invention, the sensor comprises two electrodes facing the dielectric material and forming a capacitor, an electromagnetic field at radio frequency being generated between said electrodes. Typically, the electromagnetic field has a frequency in the range from 0.1 to 200 MHz.

With an arrangement of this type it is possible to detect both surface defects and deep defects. According to the type of defect which is to be identified, the device may comprise a type of sensor suitable for the identification of surface defects, or a type of sensor suitable for the identification of deep defects, or both.

To detect surface defects, according to a possible embodiment of the capacitive sensor, the sensor has two electrodes arranged so that they face the same face of the dielectric material, in such a way that the electromagnetic field generated by the two electrodes has field lines which pass through a surface thickness of the dielectric material, starting from one of the electrodes and terminating on the other.

If defects within the thickness of the dielectric material, in other words deep defects, are to be detected, the capacitive sensor may have (according to an advantageous embodiment) two electrodes arranged respectively on two opposite faces of the dielectric material, in such a way that the electromagnetic field generated between them has field lines which pass through the whole thickness of the dielectric material between the two opposite faces which the electrodes of the sensor face.

Further advantageous characteristics of the sensor and of the device according to the invention are indicated in the attached claims.

The method of detecting the presence of defects in a dielectric material (particularly wood) has the following steps, according to the invention:

- generating an electromagnetic field in a controlled volume between two electrodes arranged close to each other;
- moving the dielectric material in the electromagnetic field in said volume;
- detecting the presence of defects in the dielectric material from the variation of the dielectric constant of said volume.

Further advantageous characteristics and embodiments of the method according to the invention are indicated in the attached claims.

The invention will be more clearly understood from the description and the attached drawing, which shows possible embodiments of the invention. More particularly, in the drawing,

Fig. 1 is a schematic diagram of a capacitive sensor for detecting surface defects;
Fig. 1A is a front view through I-I in Fig. 1;
Fig. 2 is a schematic longitudinal section through one embodiment of the sensor shown in Fig. 1;
Fig. 3 is a side view of the sensor shown in Fig. 2;
Fig. 4 is a diagram of the device for feeding the sheets of wood on which the investigation is to be performed;
Fig. 5 is a diagram of a set of sensors for detecting deep defects;
Fig. 6 is a diagram of the arrangement of an array of sensors;
Fig. 7 is a diagram of a feedback oscillator usable in combination with the sensor according to the present invention;
Fig. 8 is a block diagram of the electronic circuit associated with the sensor;
Fig. 9 is a schematic diagram showing the effect of the distance between the sensor and the dielectric material on the signal detected;

Fig. 10 is a longitudinal section through the layout of an improved sensor for detecting surface defects;

Fig. 11 is a front view through XI-XI in Fig. 10;

Fig. 12 is a diagram showing the operation of the sensor shown in Fig. 11;

Fig. 13 is a schematic diagram of an improved sensor for detecting deep defects;

Figs. 14 and 15 are schematic diagrams showing the possibilities of focusing the electromagnetic field obtainable with the sensor shown in Fig. 13; and

Figs. 16 and 17 are the experimental results of investigations carried out with a device according to the invention.

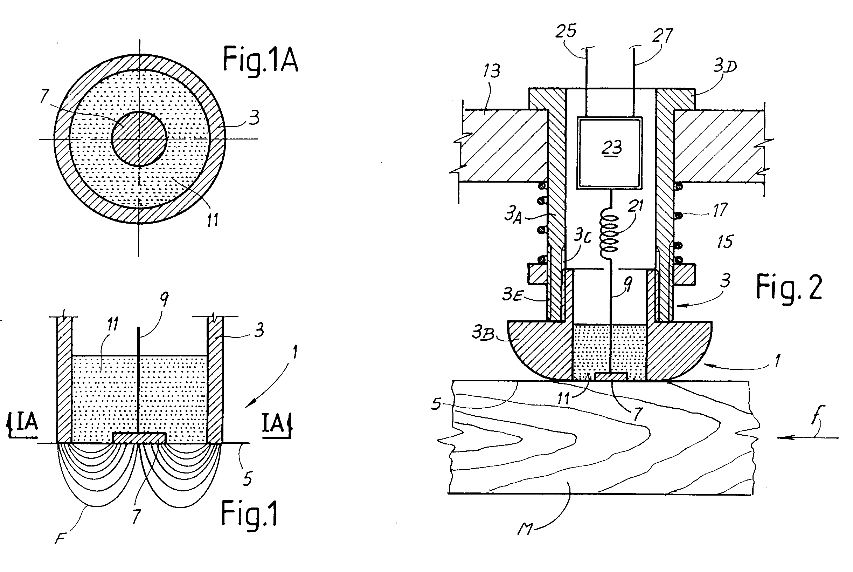

A first type of sensor, usable in the device according to the invention for identifying surface defects, will be illustrated in the first place with reference to Figs. 1 to 4. Fig. 1 shows a schematic embodiment of the sensor, indicated in a general way by the number 1. In practice, the sensor consists of a coaxial cable which has an outer conductor 3 terminating at a head plane 5, on which a metal plate 7 is respectively located. The outer conductor 3 is connected to ground. The metal plate 7 is positioned coaxially with respect to the outer conductor 3 and is connected to an inner conductor 9 which, together with the outer conductor 3, forms the coaxial cable proper. A dielectric insulator 11 is located between, on the one hand, the metal plate 7 and the internal conductor 9 associated with it and, on the other hand, the outer conductor 3. The conductors 3 and 9 are connected to an oscillator to generate an electromagnetic field between the outer conductor 3 and the plate 7. The electromagnetic field is formed by lines of force F shown schematically in Fig. 1, which extend on the opposite side of the head plane 5 from the position of the conductors 3 and 9 and the plate 7.

As will be made clear subsequently, the dielectric material, particularly wood, on which the non-invasive investigation is to be conducted to detect the presence of defects is brought to the head plane 5 of the sensor and made to slide in front of it, in such a way that the lines of force of the electromagnetic field generated between the metal plate 7 and the outer conductor 3 (forming two electrodes of a capacitor) pass through the dielectric material. When the wood is moved in front of the sensor 1, the variation of the local dielectric constant of the wood due to the presence of any defects essentially causes a variation of the overall dielectric constant of the sensor 1, detectable by means of a suitable electronic circuit which will be described subsequently.

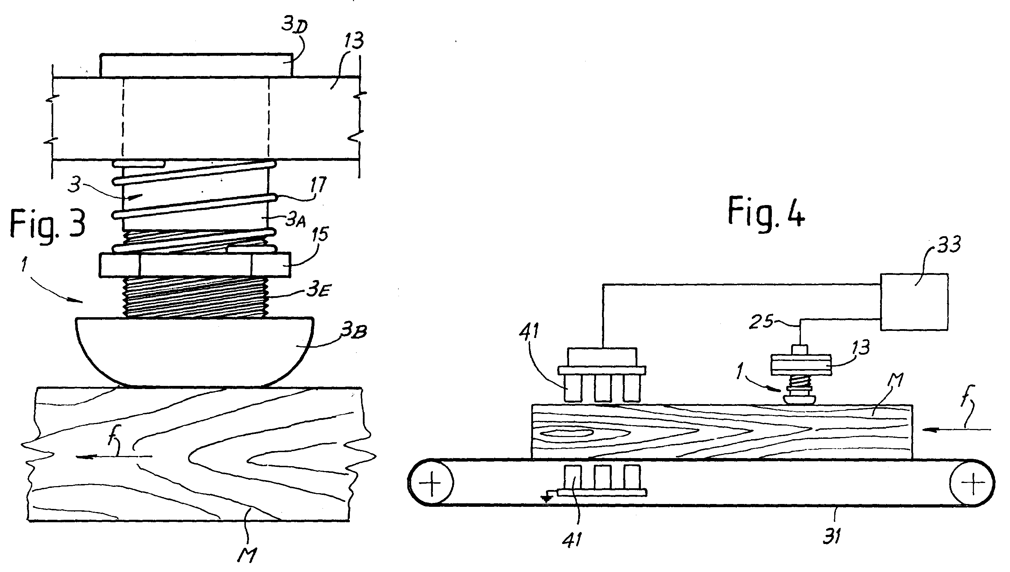

Figs. 2 and 3 show, in longitudinal section and in a side view, a practical embodiment of the sensor shown schematically in Figs. 1 and 1A. Identical numbers indicate parts corresponding to those shown in the schematic illustration in Figs. 1 and 1A. The metal plate 7 is connected to the inner conductor 9 and is surrounded by the outer conductor indicated in a general way by 3. The latter is made, in this embodiment, in two parts indicated by 3A and 3B respectively. The two parts 3A and 3B are connected together by a screw fastening 3C. The lower part 3B is rounded perimetrically for the purposes described below. The part 3A of the hollow outer conductor 3 terminates in a collar 3D supported on a fixed structure 13. A nut 15 is screwed onto an outer thread 3E. A compression spring 17, which pushes the sensor 1 downward, bringing the collar 3D to bear on the structure 13, is placed between the fixed structure 13 and the nut 15. There is no reason why the coil spring illustrated should not be replaced by another elastic element, for example a strip; or why the elastic element should not be omitted completely and the sensor brought to the bearing position by its own weight.

When the wood (or other dielectric material) indicated in a general way by M is fed under the sensor 1 in the direction of the arrow f, the head plane 5 of the sensor 1 is held against the wood M by the force of the compression spring 17. Any variations in the thickness of the wood M are compensated by a greater or lesser compression of the spring 17 which allows the sensor to move vertically. The rounded profile of the lower part 3B of the outer conductor 3 enables the wood M to be inserted easily under the sensor 1, avoiding the risk of impact and jamming.

Fig. 2 also shows schematically an inductance 21 located between the conductor 9 and an electronic circuit 23, whose functions and structure will be described below. The number 25 indicates the output of the signal (analog or digital) generated by the electronic circuit 23, while 27 indicates the power supply of the electronic circuit 23. The inductance 21, together with the capacitor formed by the plate 7 and the outer conductor 3, form an oscillating circuit whose oscillation frequency depends on the dielectric constant of the wood M, since the lines of the electromagnetic field emerging from the plate 7 pass through the wood M. The local variation of the dielectric constant of the wood M is thus detected in the form of a variation of the impedance of the capacitor formed by the electrodes 3 and 7.

In practice, a plurality of sensors 1, is arranged on the fixed structure 13, each of which can be moved vertically owing to the compressibility of the spring 17, independently of the adjacent sensors, so that any variations of thickness or warping of the sheet of wood M passing under the sensors 1 can be followed.

Fig. 4 shows in a highly schematic way a conveyer 31 on which rests a sheet M of wood which passes under the fixed structure 13, for example a beam lying transversely with respect to the direction of advance f, on which a plurality of aligned sensors 1 is arranged. The sensors 1 are connected by the lines 25 to a central unit 33, for example a personal computer.

The spatial resolution of the sensor is determined by three factors:

*a) The size of the sensor*: the sensors have a specific area of contact with the wood; the effect generated by the presence of defects with smaller dimensions is averaged over this area, and therefore if a defect is to be clearly visible it must occupy a significant percentage. At the present time, a sensor diameter of approximately 10 mm is considered satisfactory. If it were necessary to improve the resolution, sensors with smaller diameters would have to be made; this would result in a diminution of the signal, a reduction of the immunity to noise and an increase in the complexity of the electronic control system. In other words, an excessive spatial resolution entails the penalty of a lower resolution in the measurement of impedance.

*b) The distance between the sampling points in the transverse direction with respect to the direction of advance of the wood*: to increase the resolution in this direction, it is sufficient to increase the number of sensors which scan a section of the material. By arranging them in inclined directions it is possible to sample points spaced apart by a distance smaller than the sensor diameter.

*c) The distance between the sampling points in the longitudinal direction*: in this direction the sampling interval is given by the product of the response time of the electronic circuit and the speed of travel of the wood. The shortening of the intervals is therefore dependent on making a sufficiently fast electronic circuit.

Points b) and C) contribute to the determination of the resolution because if the edge of a defect is in an area intermediate between two measurement points, the effect due to this presence is divided between the two corresponding measurements. The result is a loss of contrast and a smoothing which affect both the surface inspection and the internal contour inspection.

It is possible to increase the spatial resolution which can be obtained by placing the sensors 1 on a line which is oblique with respect to the direction of travel f of the wood M. If this arrangement is adopted, the data obtained from the set of sensors must be synchronized with each other while taking the speed of advance of the wood into account. This is because the points belonging to a single cross section of the sheet do not all pass simultaneously under the sensor which measures its dielectric constant.

The sensor described up to this point is illustrated in Figs. 1 to 3 and is capable of detecting the presence of surface defects, since the lines of force F of the electromagnetic field generated between the electrodes 3 and 7 pass through these defects. When defects within the thickness of the sheet of wood are to be detected, use is made of a sensor which can be described schematically as a capacitor with parallel flat faces, whose two electrodes are arranged facing each other on the opposite faces of the sheet. The lines of force of the electromagnetic field generated between the two electrodes are enclosed between one electrode and the other, passing through the space between them. The impedance of the capacitor therefore depends on the dielectric constant of the material located between the two electrodes. If the two electrodes are at a distance approximately equal to the thickness of the sheet of wood M to be analyzed, and the latter slides between them, the measured impedance changes with the variation of the chemical and physical characteristics of the whole thickness of the dielectric medium which passes, instant by instant, through the volume between the two electrodes.

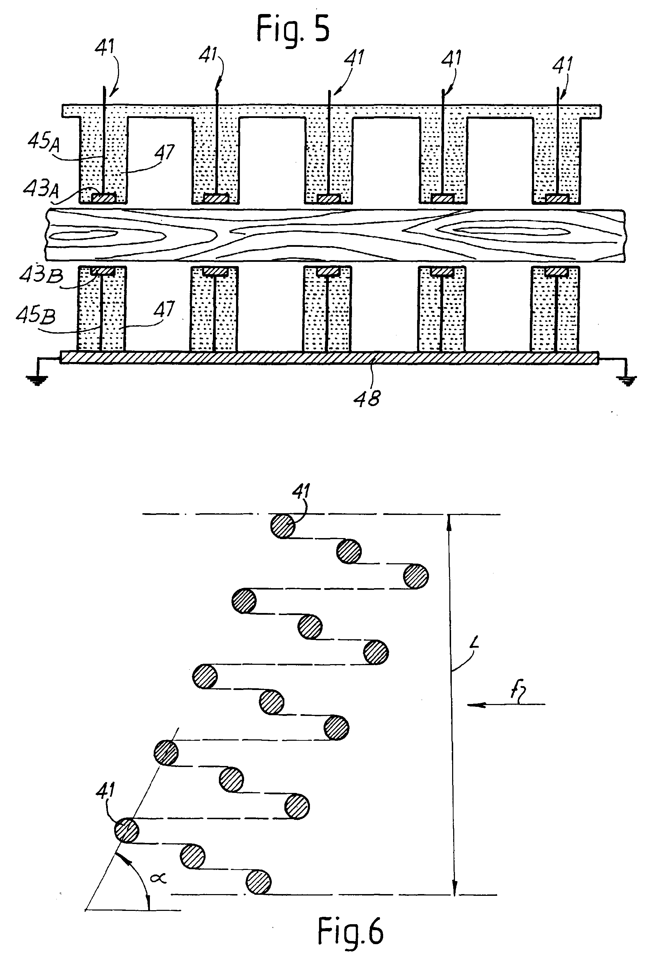

Fig. 5 shows schematically an arrangement of five sensors for detecting deep defects. Each of these sensors, indicated individually by the number 41, consists of a capacitor with flat parallel faces whose electrodes are indicated by 43A and 43B. Each electrode is carried on a corresponding column of insulating material 47. The size of each electrode 43A, 43B is relatively small, so that the individual sensors 41 are at a considerable distance from each other, in such a way that the lines of force enclosed between the electrodes of adjacent sensors are reduced. The insulating material 47, made from Teflon$^{\circledR}$ or other equivalent material, is placed around each electrode 43A, 43B and the corresponding conductor 45A, 45B.

The conductors 45B, and consequently the electrodes 43B, are connected to a common ground plane 48, so that they are approximately at the same potential, thus reducing to a minimum the number of lines of force enclosed between the electrodes of adjacent sensors. The distance between the electrodes 43A and 43B of each capacitor 41 is approximately equal to the thickness of the sheet of wood M.

In practice, a plurality of sensors 41 is arranged in a rectangular array, as illustrated in Fig. 6, where an array of 3 x 5 sensors 41 is shown. These sensors are aligned in an alignment inclined at an angle a with respect to the direction of advance f of the material M with respect to the sensors. Thus, as clearly shown in Fig. 6, the whole width L of the material M can be covered by using sensors 41 whose electrodes 43 are of a small size. The angle $\alpha$ is equal to approximately 27°.

To reduce the interference of each sensor 41 with those nearest it, use may also be made of a more complex system for reducing the distance between the sensors. This system consists in laterally covering the columns carrying the electrodes 43A with a metal screen and in connecting all the screens of the corresponding electrodes 43A to the same potential.

Fig. 4, showing the device as a whole, indicates schematically the presence of an array of sensors 41 located after the sensors 1 and connected to the same processing unit 33. With this arrangement, it is possible to carry out a superficial investigation by means of the sensors 1 and a deep investigation by means of the sensors 41.

In tests which were conducted, it was observed that the measurements in transmission (in other words the depth measurements by means of the sensors 41) were much less sensitive to the variation of the distance between the electrodes and the surface of the wood M than were the surface measurements made with the sensors 1. In general, the distance has virtually no effect, provided that it is much less than the thickness of the wood M on which the measurement is carried out.

The electromagnetic field at radio frequency which is generated between the electrodes of the sensor 1 may have a different frequency from that used between the electrodes of the sensors 41. In practice, the frequencies vary from 0.1 to 200 MHz. Optimal operating frequencies are in the area of 90-100 MHz. It is also possible to use a plurality of frequencies for each type of sensor, since some types of defect can be detected more effectively with frequencies that are ineffective for other types of defect. By means of convenient experimental tests, a person skilled in the art can conveniently identify the optimal frequencies for the various types of defect that may be encountered in the material.

A device using the sensors 1 and/or 41 has particularly advantageous characteristics for use on an industrial scale. Of these advantages, the following may be noted:

- the method can be used both for surface analysis and for internal analysis;
- the measurements are unaffected by various sources of noise, namely the degree of surface finish of the wood, dust, and dirt;
- the response of the sensors is fast and is available in a form which can be processed easily;
- the information supplied by each sensor can be used to measure various characteristic values of the wood simultaneously;
- the individual sensor is adaptable to various requirements and can easily be constructed at low cost;
- the system can be included in a line for automating production processes: a machine can be placed after the system of sensors to reject, by cutting off, the defective portions of the wood according to the measurements obtained from the sensors;
- the system constructed in this way can also be used for diagnosing the state of preservation of works of art.

The data supplied by the system of sensors described above can also be used for measuring the geometrical regularity and moisture content of wood.

To measure the dimensions, and consequently the regularity, of the sheets of wood M which pass through a system of sensors 1, 41, it is possible to proceed as follows: the sensors 41 are arranged in such a way as to form two arrays in a direction transverse with respect to the direction of advance shown by the arrow f. A first array of sensors is positioned above the upper surface and one is positioned below the lower surface of the sheet of wood M. The sensors facing each other across an area occupied by the material will give readings which differ greatly from each other. As a result of this arrangement and the advancement of the sheets, the profiles of the edges are followed and the irregularities of the surface are identified.

The electromagnetic radiation passing through a portion of the wood undergoes a variation with respect to amplitude and phase. This variation depends on certain factors, namely the thickness of the material which constitutes the kind of wood in question, the temperature, and the residual moisture present in the material. The temperature and thickness can easily be monitored in real time, and the kind of wood being processed can be pre-set by the operator on the control unit 33. By appropriate calibration of these parameters, therefore, the moisture content of the specimen can be found immediately by following the amplitude and phase variations of the signal transmitted by the sensors.

The electronic systems which complement the sensor carry out three basic tasks:

- controlling the sensor
- reading the data
- decoding

As has been repeated a number of times, the system detects the non-uniformities of the wood by means of the variations of capacitance of a capacitor; alternatively, since the sensors operate at radio frequency, we may speak in terms of variations of inductance. In this case the primary task of the electronic circuit will be to generate electrical signals proportional to these variations.

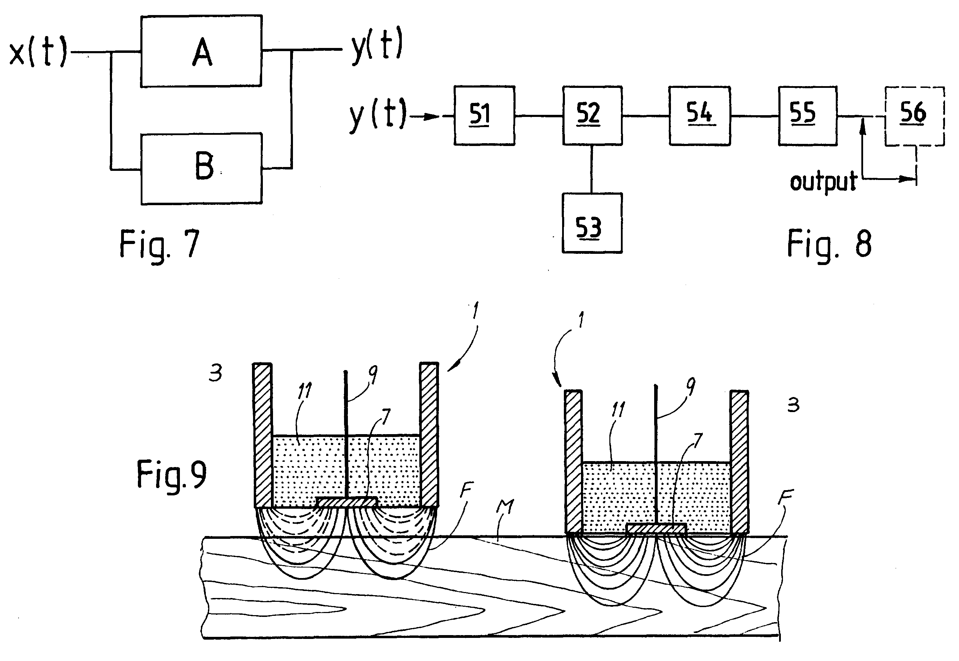

High-frequency oscillators are used to measure a variation of inductance. Fig. 7 shows a schematic diagram of an oscillator of this type. The upper box represents an amplification stage characterized by a gain A; the lower box represents the positive feedback branch, characterized by a transfer function $\beta$. The following relation is true:

$$(t) = A_r x(t) \tag{1}$$

where

x(t) = input signal

y(t) = output signal

Ar = feedback gain.

It is known from theory that the gain of the feedback amplifier is given by:

$$A_r = \frac{A}{1-A\beta} \tag{2}$$

where the minus sign in the denominator allows for the fact that the feedback is positive. If an output signal y(t) is to be present even in the absence of an input signal (x(t)=0), it is necessary that:

$$A_r \to \infty$$

and therefore

$$A\beta = 1 \tag{3}$$

The condition expressed by the preceding formula is called the Barkhausen oscillation criterion.

In general, the product $A\beta$ is a complex number, and therefore, according to (3),

$$\text{Re}(A\beta) = 1 \tag{4, a}$$

$$\text{Im}(A\beta) = 0 \tag{4, b}$$

Since $\beta = \beta(\omega)$, the condition (4,b) is met at a particular frequency:

$$\text{Im}(A\beta(\omega 0)) = 0 \tag{4, c}$$

and therefore the system oscillates at the frequency $\omega 0$ which meets the condition (4, c).

If the feedback branch undergoes a physical change due to external causes, its transfer function changes. After a brief transient, the system becomes stabilized at a new oscillation frequency $\omega 1$ such that:

$$\text{Im}(A\beta(\omega_1), = 0$$

In practice, oscillators consist of an operational amplifier with positive feedback; the oscillation frequency is established by the impedance of the feedback branch.

In the driver circuit of each sensor, the capacitance to be measured is located within the feedback branch itself and therefore any change causes a variation of frequency. If $\omega_s$ is the oscillation frequency and $Z_r$ is the impedance of the sensor,

$$\omega = (\omega(Z_r) \text{ and } \qquad Z_r = Z_r(C)$$

where C is the capacitance, which in turn depends on the dielectric constant of the material which is inserted in the volume subject to the electromagnetic field generated between the electrodes of the capacitor of the sensor. Consequently, the defects to be detected are related to a variation of the dielectric constant of the material. This is reflected in a variation of the capacitance C of the capacitor when the material is located in the volume through which the lines of force of the electromagnetic field generated between the electrodes pass, and consequently in a variation of the impedance Z of the sensor and therefore of the resonant frequency $\overline{\omega}$. In relation to the present description and the attached claims, therefore, when reference is made to the detection of the value of the dielectric constant as a parameter for detecting the presence of defects, it is to be understood that this detection can be indirect and obtained by means of a measurement of frequency or impedance.

To increase, $\frac{\delta\omega}{\delta C}$ in other words to ensure that the variations of capacitance are accompanied by large variations of the frequency $\omega$, a variable inductance is inserted in series with the capacitance represented by the electrode of the sensor. By varying the value, the feedback branch is made to resonate at $\omega_s$. If the branch has a sufficiently high quality factor Q, a small variation of capacity causes a large variation of the impedance $Z_r$, and therefore also causes a considerable displacement of the characteristic oscillation frequency.

The variations of frequency can be measured with the simple circuit described below with reference to Fig. 8.

Let y(t) be the output signal of the oscillator; the stage 51 is an adaptation stage which adapts the amplitude of y(t) to the TTL standard without changing its frequency; 52 is a switch which can permit or interrupt the passage of the signal to the box 54 which is a counter. The opening of the switch 52 is regulated by a control circuit 53. In practice, the circuit 53 may be a monostable or a counter which determines the time slot during which the switch 52 permits the passage of the signal.

To measure the frequency, the opening time of the switch 2 is set and the oscillations of the signal during this time are counted.

The box 55 is a memory which retains the data element until it is updated by the next count. Finally, the box 56 is an optional digital/analog converter, by means of which the digital data element can be converted to an analog signal so that it can be transmitted more rapidly to a control unit located at a short distance from the sensors.

According to the above description, each sensor makes available at its output a signal proportional to the quantity measured. Two methods of downloading the data may be used:

- If the output of the sensor is of the analog type (when the digital/analog converter 56 is present), each output is connected directly to the decoding station where the various inputs are controlled by a multiplexer. The system is simple and fast, but the station must be close to the sensors to avoid interference.
- If the output is digital (without the D/A converter 56), data transmission modules of the IEE485 type are used. The system is slower and somewhat more complex in terms of the electronics, but enables data to be transmitted even over a long distance.

The signals from each sensor are sent to a decoding stage (not shown). Signals proportional to the impedance measured by each sensor arrive at this stage. The output of the sensors may be different, even if they are analyzing a material with the same characteristics. This is due to small differences in design between sensors, but the effect on the measurement can be eliminated simply by calibration.

Each signal from each individual sensor can be considered as the sum of a base signal corresponding to the wood without defects and a superimposed signal, caused by the presence of a defect. This additional signal has a value which can vary according to the type of defect. The absence of material can also be considered as a defect and will give rise to a specific signal. The following may be identified:

$$S_0 = \text{signal corresponding to the wood without defects}$$

$$S_1 = S_0 + \Delta_1 = \text{signal corresponding to the absence of material}$$

$$S_2 = S_0 + \Delta_2 = \text{signal corresponding to the presence of a knot}$$

$$S_3 = S_0 + \Delta_3 = \text{signal corresponding to the presence of a pocket of resin}$$

$$S_n = S_0 + \Delta_n$$

These data can be processed by two methods:

*a) With fixed thresholds*: A range of variation for the signal S is chosen; the range is fixed; the data are classified according to their location in this range. This method can be implemented with a simple electronic circuit (of the multi-channel type) which is very fast. However, the problems arise from the fact that the signals $S_i$ depend not only on the defects but also on the thickness of the sheets or boards of wood, on the kind of wood, on the moisture content and on the temperature. Before classifying the data, therefore, it is necessary to make corrections which are stored in a database and relate to the operating conditions. All of this complicates the electronics and slows down the system.

*With variable thresholds.* It is assumed that a line of N sensors is used to inspect a sheet; while the sheet slides under the sensors, the data relating to the first n cross sections are acquired; according to the speed of sliding and the rate of sampling, n sections correspond to a certain length of material (which could also be the length of the whole sheet). The data read by the line of sensors are sent to a memory to wait until n x N of them have accumulated. The storage process must retain the characteristic spatial resolution of the collected data, in such a way that a map of the board is reconstructed in the memory. If the examined length to which the accumulated data correspond is high with respect to the dimensions of the defects, the value which appears most frequently among the n x N values read will be $S_0$, in other words that relating to the wood without defects. When $S_0$ has been found, the defects are recognized by the differences from this value. The electronic decoding circuit accesses the stored data twice, firstly in order to determine the value which appears most frequently, so that the value $S_0$ can then be determined; and secondly in order to compare the acquired data with this value $S_0$ and therefore to determine the existence and type of defects.

The principal advantages of this second method of decoding are as follows:

1. It is only slightly affected by the variations of moisture content from one sheet to another. This is because the value $S_0$ is recalibrated before each decoding, in other words whenever a new sheet of material is analyzed.
2. It is independent of the thickness of the material.
3. It is independent of the kind of wood being processed.
4. It is not affected by variations of ambient temperature.
5. It is not affected by the thermal drift of the electronic components.

The advantages 1) 2) and 3) are due to the fact that the value $S_0$ is recalibrated before each decoding; the advantages 4) and 5) are due to the fact that the thermal drifts occur over periods which are long with respect to the sheet analysis time.

In fact, the values of the signals $\Delta_i$ also depend on the moisture content, temperature and type of wood, but the effect is of a higher order than the dependence of $S_0$.

The performance of the sensors described above can be enhanced by a series of improvements. In the following text, two types of improved sensors having better performance will be described with reference to Figs. 9 to 15, one type being used for detecting surface defects and one for detecting deep defects.

The sensors described with reference to Figs. 9 to 15 were designed to improve the performance of those described previously; in particular, the objectives were:

- to decrease the sensitivity of the surface measurements to small variations of the distance between the head of the sensor and the specimen being examined;
- to screen the sensors to control internally both the reciprocal effect and the effect due to external factors.

In sensors for surface inspection, the variations of the output signal are due to the field lines which are enclosed between the inner electrode and the ground, passing through the dielectric medium under examination. As is shown clearly in Fig. 9, where two sensors identical to those in Fig. 1 are illustrated in two different positions with respect to the wood material M to be analyzed, if the sensor 1 is located at a certain distance from the medium under examination (on the left in Fig. 9), a certain number of field lines (shown as broken lines) start to be enclosed in the air. Consequently the part of the output signal due to these lines does not provide information on the medium, and reduces the sensitivity of the sensor. Conversely, in the position shown on the right in Fig. 9 all the lines of force F pass through the dielectric material consisting of the sheet M of wood.

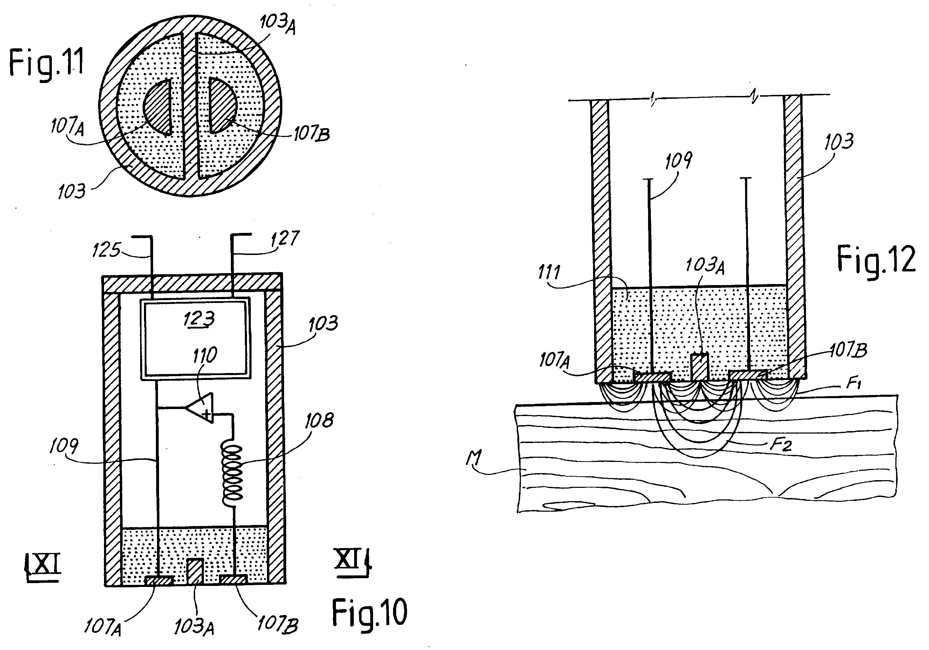

When this is considered, it is clear that any non-constant distance between the sensor and the material M to be analyzed has a significant effect on the outcome of the measurement. To reduce or eliminate this problem, it is possible to make a sensor with a structure of the type shown in Figs. 10 and 11. In this case also, the sensor, indicated in a general way by 101, has an outer conductor, indicated by 103. It externally surrounds two electrodes consisting of plates 107A and 107B. These are screened from each other by a dividing partition which consists of said outer conductor 103 and is indicated by 103A. The outer conductor 103, and therefore also the separating partition 103A, are connected to the ground. The electrode 107B is connected to the electrode 107A through an inductance 108 and an operational amplifier 110. The electrode 107A is connected to the electronic circuit 123 by a conductor 109. The numbers 125 and 127 indicate the signal output of the sensor 101 and the power supply to the electronic circuit 123. The number 111 indicates the dielectric which insulates the two electrodes 107A and 107B and the outer conductor 103.

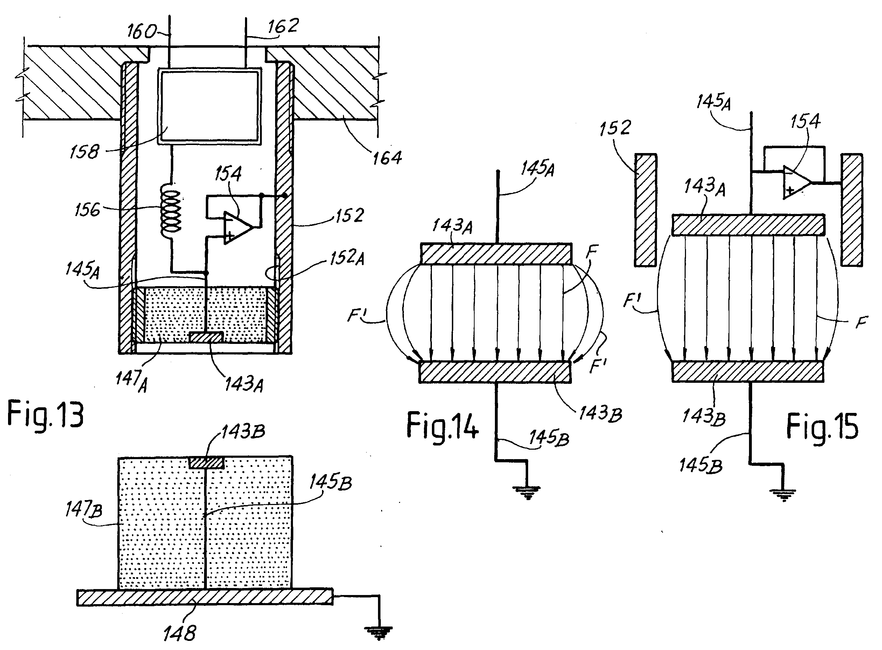

Fig. 13 shows schematically the electromagnetic field at the head of the sensor 101, using the lines of force F of the field. F1 indicates lines of force which emerge from the electrodes 107A, 107B and terminate on the outer conductor 103, 103A. F2 indicates the lines of force which are enclosed between the two electrodes 107A and 107B. It may be seen that:

- As the length of the path of the lines of force F2 linking the two electrodes increases, the percentage of signal transmitted between them decreases;
- if the lines pass through a dielectric medium, the effect of the medium becomes more significant as the percentage of the path of the lines of force running through the said dielectric medium increases.

As may be seen in Fig. 12, the presence of ground planes consisting of the outer conductor 103, 103A around and between the electrodes 107A, 107B complicates the path of the lines of force of the electromagnetic field. In this sensor, only the lines of force F2 contribute to the variations of the output signal. These lines of force follow a longer path and therefore generate a signal with a smaller amplitude; for the same reason, however, they are completed by penetrating more deeply into the wood M which is to be analyzed. If the surface of the material M under examination is moved away from the head plane of the sensor, the lines of force F1 begin to pass completely or to a large extent through the air. For the lines of force F2, however, only a small percentage of the path is in the air, and the larger part of these again passes through the material M under examination.

From these observations it may be seen that the sensor 101 generates an output signal which is weaker, although it can be electronically amplified, but which has a lower sensitivity to small variations of the distance between the probe and the surface of the wood.

The sensor 101 can be used in place of the sensor 1 when it is acceptable to have an output signal of lower intensity (or when the cost of its amplification is not significant), but it is desirable to eliminate or considerably reduce the effect of a non-constant distance or a movement of the plane of the material M to be analyzed away from the sensor.

The structure of the sensors for detecting deep defects may also be improved in order to make the interference of each sensor with the others and with any other noise source as small as possible. To achieve this purpose, use may also be made of screening metal surfaces. An example of an improved sensor of this type is shown in Fig. 13 and is indicated in a general way by the number 141. It has two electrodes 143A and 143B, forming two electrodes of a capacitor with flat parallel faces, similar to the electrodes 43A and 43B in Fig. 5. The electrode 143A is connected by a conductor 145A to an electronic circuit described below, while the electrode 143B is connected by a conductor 145B to a plate 148 held at ground potential. The numbers 147A and 147B indicate blocks of insulating material into which the conductors 145A and 145B and the electrodes 143A and 143B are inserted. The block 147A, within which the electrode 143A is housed, is screwed into a thread 152A of an outer tubular metal screen 152, in such a way that it can be inserted to a greater or lesser degree, together with the electrode 143, into the screen 152 itself.

The electrode 143A is connected electrically to the outer screen 152 through an emitter follower 154 for the purposes stated below. The electrode 143A is also connected through an inductance 156 to an electronic circuit 158, which has a signal output 160 and a power supply line 162. With this arrangement, the tubular metal screen 152, which is mounted on a fixed structure (not shown) by means of an electrically insulating element 164, made from Teflon® for example, is insulated from the ground and is held at the same potential as the electrode 143A by the action of the emitter follower 154. The emitter follower performs this function without any feedback to the circuit to which it is connected.

From field theory, it is known that there cannot be any line of force which links points at the same potential, and therefore the presence of the tubular metal screen 152 does not affect the measurement in any way. At the same time, however, the tubular metal screen 152 protects the electrode 143A from interference. This is because the presence of external electromagnetic fields only causes an increase in the work done by the emitter follower 154.

Because the electrode 143A can be inserted into the outer tubular screen 152, it is possible to obtain a further advantage, illustrated with reference to Figs. 14 and 15. If the other contributions are disregarded, the electromagnetic field between the two electrodes 143A and 143B shown schematically in Figs. 14 and 15 consists of lines of force F which link the electrodes 143A, 143B, passing through the area which in geometrical terms lies between them and the lines of force F' passing through adjacent areas.

In the configuration in Fig. 15, the path between the two electrodes has been increased with respect to the configuration in Fig. 14, and this decreases the amplitude of the working signal. At the same time, however, there is a much greater decrease in the edge effect which decreases the definition of the images produced. This is because the number of lines of force F' which emerge from the volume lying between the two electrodes decreases considerably with a change from the configuration in Fig. 14 (without a screen) to the configuration in Fig. 15 (with an outer tubular screen 152 and the electrode 143A partially inserted in it). In other words, the radio frequency radiation is focused in this way.

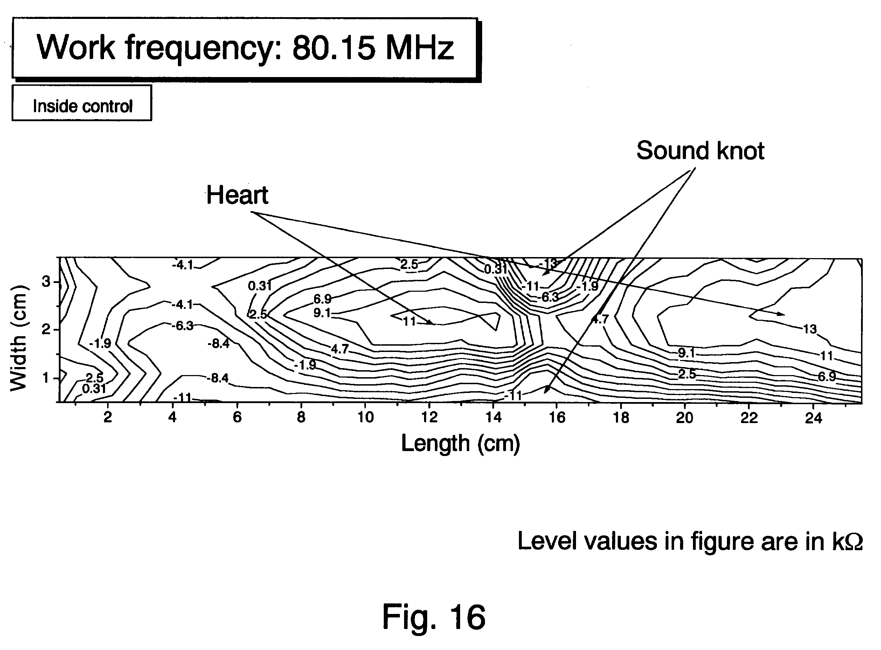

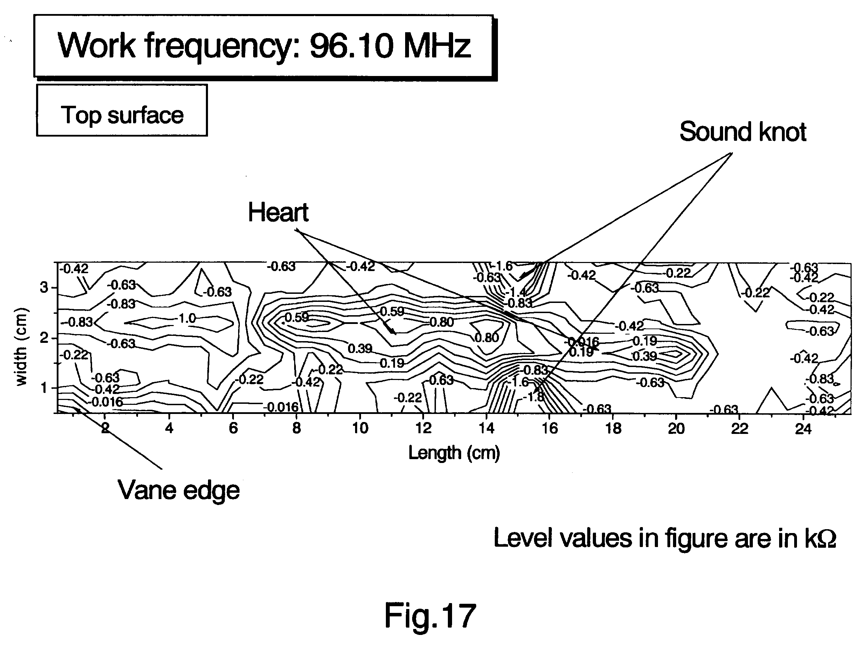

Figs. 16 and 17 show in a plan view the result of an investigation conducted on a single specimen of wood with a set of sensors 41 for internal analysis and with a set of sensors 1 for surface analysis respectively. Each figure shows the level curves corresponding to various values of measured impedance, corresponding to the presence of various types of defect. The deep defects were detected at an operating frequency of 80.15 MHz, while the surface defects were detected at an operating frequency of 96.10 MHz. Observation of the two images reveals the presence of a central heart which comes to the surface in the central area of the piece, as shown in Fig. 17, at the position of the level curves having the highest impedance value. The image in Fig. 16 shows the presence of the same heart in the central position, but also impedance values of the same order of magnitude in the right-hand area of the image. This is due to the fact that the defect comes to the surface in the central area and then extends inside the piece, penetrating into its interior. It is therefore visible in the right-hand area only in the deep examination (Fig. 16).

Also in the central area, two knots are detected near the longitudinal edges, being characterized by very low impedance values. The knots are detected in both images, since they come to the surface. However, the deep image (Fig. 16) shows a more marked defect along the upper edge. This is due to the fact that the piece actually had a second knot at a greater depth than the first and underneath the first. This produced a larger signal in the deep image than in the surface image.

Figs. 16 and 17 were obtained with two sets of sensors, the piece being analyzed in depth and on one surface only. In practice, it is possible to provide a double set of surface sensors in the same device, to detect the surface defects on both faces of the piece. Additionally, the signals obtained from the two or three sets of sensors can be combined to provide a combined signal.

It is to be understood that the drawing shows only some embodiments of the invention, which can be varied in its forms and arrangements without departure from the scope of the underlying concept of the invention. The presence of any reference numbers in the following claims has the sole purpose of facilitating the reading of the claims with reference to the preceding description and to the attached drawings, and does not limit their scope of protection.

**Claims**

1.  A device for detecting defects in a dielectric material, particularly wood, comprising at least one capacitive sensor, means for placing said capacitive sensor next to the dielectric material, and a detection circuit for detecting the presence of defects in said material according to the variations of the value of the dielectric constant detected by said sensor.

2.  Device as claimed in claim 1, wherein said at least one sensor comprises two electrodes facing said dielectric material and forming a capacitor, an electromagnetic field at radio frequency being generated between said electrodes.

3.  Device as claimed in claim 2, wherein said electromagnetic field has a frequency in the range from 0.1 to 200 MHz.

4.  Device as claimed in claim 2 or 3, wherein said two electrodes of said at least one sensor face the same face of the dielectric material, said electromagnetic field having field lines which pass through a surface thickness of said dielectric material.

5.  Device as claimed in claim 2 or 3, wherein said two electrodes of said at least one sensor are respectively arranged on two opposite faces of the dielectric material, said electromagnetic field having field lines which pass through the whole thickness of the dielectric material between the two opposite faces which the two electrodes of said at least one sensor face.

6.  Device as claimed in claim 2 or 3, comprising:

    -   at least one sensor comprising two electrodes facing one face of said dielectric material, the electromagnetic field generated between said two electrodes having field lines which pass through a surface thickness of said dielectric material;
    -   at least one additional sensor having two electrodes facing two opposite faces of the dielectric material, between which is generated an electromagnetic field whose field lines pass through the whole thickness of said dielectric material between said two opposite faces.

7.  Device as claimed in claim 4 or 5, comprising a plurality of said sensors arranged in an array.

8.  Device as claimed in claim 6, comprising a plurality of said first sensors arranged in a first array, and a plurality said additional sensors arranged in a second array.

9.  Device as claimed in claim 4, 6, 7 or 8, wherein said at least one sensor comprises a small central plate forming a first electrode, an annular conductor surrounding said plate and forming a second electrode, and a dielectric between said plate and said annular conductor.

10. Device as claimed in claim 9, wherein said annular conductor forms a sliding guide to enable the sensor to bear on the dielectric material.

11. Device as claimed in claim 4, 6, 7 or 8, wherein said at least one sensor comprises a pair of electrodes forming said two capacitor electrodes, said two electrodes being surrounded and screened from each other by a conducting screen held at ground potential.

**12.** Device as claimed in one or more of claims 9 to 11, wherein said at least one sensor is supported movably on a carrying structure.

**13.** Device as claimed in claim 12, wherein said at least one sensor is pushed elastically toward a working position by an elastic member associated with said structure.

**14.** Device as claimed in claim 5, wherein at least a first electrode of said at least one sensor is surrounded by a screening conductive wall.

**15.** Device as claimed in claim 14, wherein said screening conductive wall has a front edge facing the dielectric material and wherein said first electrode is placed in a withdrawn position with respect to said front edge.

**16.** Device as claimed in claim 15, wherein the position of said first electrode with respect to said front edge is adjustable.

**17.** Device as claimed in one or more of claims 14, 15 or 16, wherein a dielectric is placed between said first electrode and said screening conductive wall.

**18.** Device as claimed in one or more of claims 14 to 17, wherein said screening conductive wall is constantly held at the same electrical potential as said first electrode.

**19.** Device as claimed in claim 18, wherein an emitter follower is placed between said screening conductive wall and said first electrode.

**20.** Device as claimed in one or more of the preceding claims, wherein said at least one sensor comprises two electrodes forming a capacitor inserted in an oscillating circuit comprising an inductance.

**21.** Device as claimed in claim 20, wherein said inductance is arranged in series with said capacitor.

**22.** Device as claimed in claim 20 or 21, wherein said capacitor and said inductance are inserted in a positive feedback system of a feedback oscillator.

**23.** Device as claimed in claim 22, wherein the output signal of the oscillator is sent to an oscillation frequency detecting circuit.

**24.** Device as claimed in claim 23, wherein said detecting circuit comprises a controlled switch, a means of controlling the opening and closing of the controlled switch, a counter and a volatile memory.

**25.** Device as claimed in claim 24, wherein said detecting circuit comprises a digital/analog converter for converting the value stored in said volatile memory into an analog value.

**26.** Device as claimed in one or more of the preceding claims, comprising means of movement for moving said dielectric material and said at least one sensor with respect to each other along a direction of movement.

**27.** Device as claimed in one or more of the preceding claims, wherein said means of movement comprise feed means for feeding said dielectric material to said at least one sensor for detecting the defects.

**28.** Device as claimed in claim 26 or 27, comprising a plurality of said sensors arranged in at least one straight line, which forms an angle different from 90° with respect to said direction of movement.

**29.** Device as claimed in one or more of the preceding claims, comprising storage means for storing a plurality of values of said dielectric constant and programmed processing means for determining the most frequent value of said dielectric constant detected in the dielectric material, and for detecting any defects in said dielectric material by comparing the stored values of said dielectric constant with a threshold value defined by said most frequent value.

**30.** A method for detecting the presence of defects in a dielectric material, comprising the steps of:

- generating an electromagnetic field in a space between two electrodes positioned close to each other;

- moving said dielectric material in said electromagnetic field;
- detecting the presence of defects in said dielectric material from the variation of the dielectric constant of said space.

**31.** Method as claimed in claim 30, wherein said dielectric material is a sheet.

**32.** Method as claimed in claim 31, wherein said sheet is a sheet of wood.

**33.** Method as claimed in claim 31 or 32, wherein said sheet passes between said two electrodes which face opposite faces of said sheet, the field lines of the electromagnetic field between said two electrodes passing though the whole thickness of the sheet.

**34.** Method as claimed in claim 31 or 32, wherein said sheet passes in front of said two electrodes which are located on the same side of the sheet.

**35.** Method as claimed in claim 31 or 32, comprising the steps of:

- arranging said at least one first and one second electrode opposite each other, facing opposite faces of said sheet to detect any defects within the thickness of the sheet;
- arranging at least one additional pair of electrodes of at least one additional capacitor adjacent to each other and facing the same face of said sheet to detect any surface defects of said sheet.

**36.** Method as claimed in one or more of claims 30 to 35, comprising the steps of:

- detecting a plurality of values of the dielectric constant of said dielectric material in a plurality of areas of the material and storing said plurality of values;
- determining the most frequent value of said plurality of values;
- detecting the presence of defects in said material according to the deviation of the stored values from said most frequent value.

Fig.2

Fig.1A

Fig.1

Fig. 3

Fig. 4

EP 1 061 361 A1

EP 1 061 361 A1

Fig. 7

Fig. 8

Fig. 9

EP 1 061 361 A1

Fig.11

103A

107A

107B

103

125

127

123

110

109

108

XI

103

XI

107A

103A

107B

Fig.10

111

109

103

103A

Fig.12

107A

107B

F₁

F₂

M

Fig.13

Fig.14

Fig.15

EP 1 061 361 A1

Fig. 16

Fig.17

EP 1 061 361 A1

**European Patent Office**

## EUROPEAN SEARCH REPORT

**Application Number**

EP 99 83 0322

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 585 732 A (STEELE PHILIP H ET AL) 17 December 1996 (1996-12-17) <br> * abstract; figures 2,3 * <br> * column 6, line 5 * <br> * column 7, line 58 - column 8, line 36 * <br> * column 11, line 49 - line 50 * <br> * column 5, line 32 * <br> --- | 1-3,5,7, 26-33 | G01N27/24 <br> G01N27/22 <br> G01N33/46 <br> G01R27/26 |
| X | GB 1 040 440 A (FLIGHT REFUELLING LIMITED) 24 August 1966 (1966-08-24) <br><br> * page 1, line 25 - line 62; figures 1-3 * <br> * page 2, line 24 - line 64 * <br> --- | 1-3,5,7, 14-19, 26,27, 30-33 | |
| X | US 4 616 425 A (BURNS STANLEY G) 14 October 1986 (1986-10-14) <br><br> * abstract; figures 1-5,8 * <br> * column 5, line 28 - line 50 * <br> * column 1, line 20 * <br> --- | 1-10,23, 24,26, 27,30-35 | |
| X | EP 0 895 081 A (TRANSTECH SYSTEMS INC) 3 February 1999 (1999-02-03) <br><br><br> * column 9, paragraph 42 * <br> * column 1, line 24 - line 25 * <br> * column 4, line 47 - column 5, line 1; figures 4-6,8 * <br> * column 5, line 20 - column 6, line 52 * <br> --- | 1-4,9, 10, 20-25, 30,34 | **TECHNICAL FIELDS SEARCHED** (Int.Cl.7) <br><br> G01N <br> G01R |
| X | US 3 671 857 A (BERGMANIS KARLIS ALFREDOVICH ET AL) 20 June 1972 (1972-06-20) <br><br> * column 1, line 7 - line 63; figure 1 * <br> --- | 1-4,9, 10, 23-25, 30,34 | |

-/--

The present search report has been drawn up for all claims

| Place of search | Date of completion of the search | Examiner |
|---|---|---|
| MUNICH | 26 November 1999 | Strohmayer, B |

EPO FORM 1503 03.82 (P04C01)

**European Patent Office**

## EUROPEAN SEARCH REPORT

Application Number

EP 99 83 0322

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 4 563 635 A (WAGNER DELMER W ET AL) 7 January 1986 (1986-01-07)<br><br>* abstract; figures 2,3 *<br>* column 4, line 32 - line 36 *<br>--- | 1,3, 20-22, 26,27, 30-32 | |
| X | US 4 972 154 A (BECHTEL FRIEND K ET AL) 20 November 1990 (1990-11-20)<br><br>* abstract *<br>* column 2, line 10 - line 11; figure 3 *<br>* column 8, line 23 - line 41 *<br>--- | 1-4, 9-11,26, 27, 30-32,34 | |
| X | US 3 793 585 A (WILSKA M) 19 February 1974 (1974-02-19)<br><br>* abstract *<br>* column 3, line 33 - line 52; figure 1 *<br>--- | 1-4,9, 10, 20-22, 30,34 | |
| X | US 5 394 097 A (ALLEN JAMES R ET AL) 28 February 1995 (1995-02-28)<br>* column 1, line 22 - line 50 *<br>* column 17, line 4 - line 37; figure 2 *<br>--- | 1,3,28, 30 | |
| X | US 3 781 672 A (MALTBY F ET AL) 25 December 1973 (1973-12-25)<br><br>* abstract; figures 1,3 *<br>* column 5, line 66 - column 6, line 6 *<br>--- | 1-6, 8-10, 14-19, 30,33-35 | |

TECHNICAL FIELDS
SEARCHED (Int.Cl.7)

-/--

The present search report has been drawn up for all claims

| Place of search | Date of completion of the search | Examiner |
|---|---|---|
| MUNICH | 26 November 1999 | Strohmayer, B |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

**European Patent Office**

**EUROPEAN SEARCH REPORT**

Application Number

EP 99 83 0322

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | DE 30 28 715 A (LAMBECK MARTIN PROF DR ING) 25 February 1982 (1982-02-25) <br> * page 3 * <br> * page 11, line 3 from bottom * <br> * claim 1 * | 1-3, 20-22,30 | |
| | ----- | | |
| | | | TECHNICAL FIELDS SEARCHED (Int.Cl.7) |

The present search report has been drawn up for all claims

| Place of search | Date of completion of the search | Examiner |
|---|---|---|
| MUNICH | 26 November 1999 | Strohmayer, B |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

## ANNEX TO THE EUROPEAN SEARCH REPORT
## ON EUROPEAN PATENT APPLICATION NO.

EP 99 83 0322

This annex lists the patent family members relating to the patent documents cited in the above-mentioned European search report.
The members are as contained in the European Patent Office EDP file on
The European Patent Office is in no way liable for these particulars which are merely given for the purpose of information.

26-11-1999

| Patent document cited in search report | | Publication date | Patent family member(s) | | Publication date |
|---|---|---|---|---|---|
| US 5585732 | A | 17-12-1996 | NONE | | |
| GB 1040440 | A | | NONE | | |
| US 4616425 | A | 14-10-1986 | NONE | | |
| EP 0895081 | A | 03-02-1999 | US | 5900736 A | 04-05-1999 |
| US 3671857 | A | 20-06-1972 | NONE | | |
| US 4563635 | A | 07-01-1986 | NONE | | |
| US 4972154 | A | 20-11-1990 | CA | 2017582 A | 06-12-1990 |
| | | | WO | 9208142 A | 14-05-1992 |
| US 3793585 | A | 19-02-1974 | NONE | | |
| US 5394097 | A | 28-02-1995 | US | 5654643 A | 05-08-1997 |
| US 3781672 | A | 25-12-1973 | US | 3860882 A | 14-01-1975 |
| DE 3028715 | A | 25-02-1982 | NONE | | |

EPO FORM P0459

For more details about this annex : see Official Journal of the European Patent Office, No. 12/82